Europäisches Patentamt

(19) European Patent Office    (11) Veröffentlichungsnummer: **0 139 706**

Office européen des brevets                **B1**

# (12)    EUROPÄISCHE PATENTSCHRIFT

(45) Veröffentlichungstag der Patentschrift:
03.02.88

(21) Anmeldenummer: **84901573.0**

(22) Anmeldetag: **05.04.84**

(86) Internationale Anmeldenummer:
**PCT/DE 84/00078**

(87) Internationale Veröffentlichungsnummer:
**WO 84/04033 (25.10.84 Gazette 84/25)**

(51) Int. Cl.⁴: **A 61 B 5/14**

(54) **TRAGBARES ENTNAHMEGERÄT FÜR BLUT UND ANDERE FLÜSSIGKEITEN UND VERFAHREN ZUR BLUT- UND FLÜSSIGKEITSENTNAHME.**

(30) Priorität: **12.04.83 DE 3313074**

(43) Veröffentlichungstag der Anmeldung:
**06.05.85 Patentblatt 85/19**

(45) Bekanntmachung des Hinweises auf die Patenterteilung:
**03.02.88 Patentblatt 88/5**

(84) Benannte Vertragsstaaten:
**AT BE CH FR GB LI NL SE**

(56) Entgegenhaltungen:
**DE-B-1 283 334**
**FR-A-1 351 154**
**US-A-4 008 717**
**US-A-4 077 395**
**US-A-4 127 111**
**US-A-4 258 717**

(73) Patentinhaber: **STEPHAN, Eberhart, Freihorstfeld 70, D-3000 Hannover 71 (DE)**

(72) Erfinder: **STEPHAN, Eberhart, Freihorstfeld 70, D-3000 Hannover 71 (DE)**

(74) Vertreter: **Junius, Walther, Dr., Wolfstrasse 24, D-3000 Hannover 81 (DE)**

## Beschreibung

Die Erfindung betrifft ein Entnahmegerät für Blut und andere Körperflüssigkeiten, bestehend aus einem Katheter, einer Pumpe, einem Vorratsbehälter, in welchem mehrere in zeitlichem Abstand gewonnene Körperflüssigkeitsproben getrennt speicherbar sind, einer Spül- und Füllvorrichtung für die Pumpe, den Katheter und ein von der Pumpe zum Speicher führendes Kanalsystem, wobei ein Ventil oder Ventilsystem für die Durchleitung der entnommenen Körperflüssigkeit und der Spül- und Füllflüssigkeit für das Kanalsystem vor dem Vorratsbehälter angeordnet ist und einer Steuervorrichtung für die Betätigung der Pumpe, der Spül- und Füllvorrichtung und dem Ventil bzw. dem Ventilsystem.

Ein derartiges Entnahmegerät ist aus der US-A-40 77 395 bekannt geworden. Dieses Gerät weist als Speicher einen Drehtisch mit einer kreisrunden Halterung für eine Vielzahl von Probenröhrchen auf, in die abwechselnd die entnommene Körperflüssigkeitsprobe und Spülmittel eingefüllt werden. Dieses Gerät weist zwei Schlauchleitungen auf, eine vom Katheter kommende und eine vom Spülmittelgefäß kommende, die jede mit einem Ventil versehen sind. Die beiden Leitungen führen in je einen der drei Anschlüsse eines Verzweigungsstückes, dessen dritter Anschluß an einen zur Pumpe führenden Schlauch angeschlossen ist. Die Pumpe befindet sich hier zwischen diesem Verzweigungsstück und einem Abfüllstutzen für die Proberöhrchen. Dieses Gerät weist eine Reihe von Schaltern auf, unter denen sich ein Programmschalter befindet. Das Gerät ist nicht tragbar. An einem Ständer ist das Spülmittelgefäß mit heparinisierter physiologischer Kochsalzlösung angeordnet, welche unter dem Einfluß der Schwerkraft bei geöffnetem Ventil in der Spülmittelleitung in das Kanalsystem eindringt.

Durch die besondere Anordnung dieses Kanalsystems und der Pumpe in diesem Kanalsystem sind für die Spülung zwei Vorgänge notwendig, ein Vorgang, bei dem das zum Katheter führende Ventil geschlossen ist und die Pumpe Spülflüssigkeit aus dem Vorratsbehälter in ein Proberöhrchen fördert, sowie ein zweiter Spülvorgang bei stillgesetzter Pumpe und geöffnetem Ventil in der zum Katheter führenden Leitung, bei dem unter dem Einfluß von Schwerkraft die Spülflüssigkeit durch die zum Katheter führende Leitung fließt. Dieses Spülen in zwei verschiedenen Arbeitsschritten ist zeitaufwendig und auch aus anderen Gründen nachteilig. Das Programm dieses Entnahmegerätes sieht vor, daß zuerst eine Blutprobe mit Hilfe der Pumpe angesaugt wird und in ein Proberöhrchen gefüllt wird, dann das Ventil in der zum Katheter führenden Leitung geschlossen wird, während das Ventil in der Spülflüssigkeitsleitung geöffnet wird und anschließend Spülflüssigkeit nach Weiterverdrehung des Drehtisches in ein gesonders Proberöhrchen eingefüllt wird. Sodann findet die schon erwähnte Stillsetzung der Pumpe in einem dritten Verfahrensschritt statt und man läßt bei geöffneten Ventilen in der Spülmittelleitung und der zum Katheter führenden Leitung Spülmittel unter dem Einfluß der Schwerkraft in den Katheter laufen. Bei diesem Programm ist es nachteilig, daß beim nächsten Ansaugen einer Blutprobe zuerst das im Leitungssystem stehengebliebene Spülmittel angesaugt und in das Proberöhrchen gefüllt wird. Man erhält somit Blutproben, die durch das Spülmittel verunreinigt sind und daher für eine Reihe von Untersuchungen nicht brauchbar sind.

Bei der mit diesen Entnahmegeräten vorgenommenen direkten Blutentnahme am Probanden wirken sich zuweilen psychische Einflüsse oder sonstige, z. B. physikalische Verhältnisse auf die Blutwerte und auf die Blutentnahme störend aus. Mit diesen Entnahmegeräten kann zwar bei den aufwendigeren Ausführungsformen eine wiederholte Blutentnahme erfolgen, diese Geräte sind jedoch nicht vom Probanden tragbar.

Im Bereich der Medizin interessieren Informationen über das Blut und seine Bestandteile und Inhaltsstoffe sowie über deren physiologische und pathologische Veränderungen. Diese Informationen können durch Gewinnung von Blutproben und deren Analyse sowie durch Einlegen von Meßfühlern und Kathetern erhalten werden. Bei der direkten Gewinnung u.ä. kann die psychische Beeinflussung des Probanden zu einer Veränderung der Blutwerte führen. Außerdem sind solche Entnahmemethoden bei manchen Untersuchungen unter Belastungsbedingungen, z. B. Lauftraining, von Rehabilitationspatienten, Sportlern u.ä., Belastungsuntersuchungen an Tieren, nur bedingt möglich, weil dazu die Belastung unterbrochen werden muß und sich die Blutwerte in dieser Zeit wieder verändern können. Bei bestimmten Belastungen, z. B. simulierten oder tatsächlichen Flugsituationen bei Piloten, Belastungsuntersuchungen von freilaufenden Tieren u.ä. ist eine solche Untersuchung überhaupt nicht möglich.

Für derartige Untersuchungen des Blutes und anderer Körperflüssigkeiten bei bestimmten Belastungen an Mensch und Tier ist ein tragbares Entnahmegerät notwendig, welches zu bestimmten je nach Situation frei wählbaren oder vorher bestimmbaren Zeiten Entnahmevorgänge so vornimmt, daß nicht mehr die Gegenwart eines die Blutentnahme vornehmenden Menschen in der Nähe notwendig ist.

Der Erfindung liegt die Aufgabe zugrunde, ein Entnahmegerät für Körperflüssigkeiten von menschlichen und tierischen Probanden so zu gestalten, dß die Blutentnahme zu beliebigen Zeitpunkten wiederholt erfolgen kann, ohne daß eine Person für die Bedienung des Entnahmegerätes in der Umgebung des Probanden tätig sein muß.

Das Entnahmegerät der vorliegenden Erfindung zeichnet sich dadurch aus, daß das Entnahmegerät am Körper eines Probanden tragbar ausgestaltet ist und daß die Steuervorrichtung durch eine Fernsteuerung einschaltbar ist.

Ein derartiges Entnahmegerät läßt sich in einem Gehäuse mit Anschnallgurt unterbringen. Durch die Fernsteuerung, die zweckmäßigerweise eine Funkfernsterung ist, ist es möglich, daß entweder gemäß einem eingegebenen Programm oder gemäß den Anweisungen einer weitab sich befindenden Person dem Entnahmegerät Impulse zugeführt werden, die zu einer Tätigkeit des Pumpenantriebes und damit zu einer Entnahme von Körperfüssigkeit und einer anschließenden Spülung des Kanalsystems der Vorrichtung und der Pumpe sowie des Katheters führen. Es ist ein besonderer Vorteil dieser Vorrichtung, daß das gesamte Kanalsystem und der Katheter ständig mit Flüssigkeit gefüllt und daher gasfrei sind. Denn die Spülflüssigkeit ist gleichzeitig Füllflüssigkeit für den Katheter. Statt Spül- und Füllflüssigkeit wird im folgenden der Einfachheit halber immer nur von Spülflüssigkeit geschrieben. In dem Gerät sind dabei im Vorratsbehälter die Körperflüssigkeitsproben in einer recht großen Anzahl speicherbar. Die hier verwendeten Arbeitsmittel lassen sich weitgehend miniaturisieren. Diese Miniaturisierung führt letzlich auch zu einer sehr kleinen notwendigen Energiequelle in Form einer elektrischen Batterie oder eines Akkumulators.

Besonders vorteilhaft ist es, wenn der Vorratsbehälter mehrere Räume für die Aufnahme von Proben aufweist, wenn vor dem Vorratsbehälter ein Mehrwegeventil angeordnet ist, bei dem je ein Weg zu je einem Raum für die Aufnahme einer Probe führt und wenn im Mehrwegeventil ein Weg für die Abführung der Spülflüssigkeit vorgesehen ist. Mit dieser Ausführungsform lassen sich z. B. bei der Beobachtung von Arbeitsprozessen eines Menschen oder eines Tieres Blutentnahmen in ständiger Wiederholung vornehmen, ohne daß der Proband es merkt und dadurch beeinflußt wird. Auf diese Weise lassen sich Rückschlüsse, z. B. auf die Einflußnahme verschiedenster Organe auf das Blut vor und während bestimmter körperlicher und seelischer Belastungen ermitteln.

Bei dieser Ausführungsform ist es zweckmäßig, wenn das Mehrwegeventil einen Schieber mit einem Kanal aufweist, dessen Mündung vor verschiedene Ausflußöffnungen einstellbar ist, von denen je eine zu verschiedenen Aufnahmebehältern und mindestens eine in einen freien Abfluß führt. Hierdurch wird das Gerät besonders leicht, weil die angesaugte Spülflüssigkeit nicht mehr in Proberöhrchen abgefüllt, sondern frei laufengelassen wird. Gleichzeitig wird hierdurch auch eine kleinere Bauform des Gerätes erzielt, die das Gerät leichter und unauffälliger tragbar macht.

Das Entnahmegerät kann aber auch einen anders gestalteten Vorratsbehälter für die Speicherung von Proben haben. Eine für die wünschenswerte Miniaturisierung des Gerätes gut geeignete Ausführung besteht darin, daß der Vorratsbehälter aus einem Schlauch oder Rohr besteht, in den bzw. das in ständigem Wechsel eine Probe der Körperflüssigkeit und eine besimmte Menge Trenngas (z. B. Luft oder ein inertes Gas), das in Form einer Blase die einzelnen Proben voneinander trennt, nach jeder Betätigung der Pumpe einleitbar ist, daß ein Vorratsbehälter für die Pufferflüssigkeit vorgesehen ist, daß ein Ventil oder ein Ventilsystem vor dem Vorratsbehälter angeordnet ist, mit dem nach jeder Probe der Körperflüssigkeit eine bestimmte Menge Trenngas in den Vorratsbehälter für die Speicherung von Proben einleitbar ist und daß ein weiteres Ventil oder Ventilsystem für die Ein- und Abführung eines Spülmittels in das Kanalsystem zwischen Pumpe und Vorratsbehälter für die Speicherung von Proben einerseits und in die Pumpe und den Katheter andererseits vorgesehen ist.

Als besonders vorteilhaft hat es sich erwiesen, daß die Steuervorrichtung für die Spülvorrichtung sowie für das dieser zugeordnete Ventil und/ oder die dem Vorratsbehälter für die Speicherung von Proben zugeordneten Ventile durch einen Impuls der Fernsteuerung in Tätigkeit setzbar ist und daß die Steuervorrichtung mit einem Programm

a in den Abfall pumpen
b umschalten
c in das Proberöhrchen pumpen
d umschalten
e spülen und in den Abfall laufenlassen

versehen ist. Denn hierdurch wird erreicht, daß in den Proberöhrchen sich reine, spülmittelfreie Blutproben befinden. Als zweckmäßig hat es sich erwiesen, wenn die Zuführungsleitung der Spülflüssigkeit zwischen dem Mehrwegeventil und der Pumpe angeordnet ist, wobei vorzugsweise die Pumpe eine Schlauchpumpe ist und wobei vorzugsweise der Spülflüssigkeitsbehälter ein flexibler gas- bzw. luftfreier Behälter ist, der bei fraktionierten Entnahmen in sich zusammenfällt. Hierdurch findet die Spülung ineinem Arbeitsgang derart statt, daß alle Teile im Leitungssystem, auch die Pumpe, einwandfrei gespült werden. Anstelle der Funkfernsteuerung kann bei ortsfestem Betrieb dieser Betrieb auch auf andere Weise, z. B. über Kabel oder - z. B. bei Benutzung einer Großzentrifuge - über Kabel und Schleifkontakte ausgelöst werden.

Über die eingebaute Steuervorrichtung ist ferner die Ansteuerung weiterer Geräte nach Wahl, z. B. Aufzeichnungs- oder Speichergeräte für physiologische o.ä. Daten, möglich.

Die mit der Erfindung erzielbaren Vorteile bestehen insbesondere darin, daß mehrfache Körperflüssigkeitsentnahmen, auch unter erschwerten äußeren Bedingungen, ohne psychische Belastung des Probanden und ohne eine weitere Person in der näheren Umgebung des Probanden möglich geworden sind.

Da die Entnahme von Körperflüssigkeit nicht unbedingt über eine Steuerungsvorrichtung durchgeführt zu werden hat, sondern auch die einzelnen Vorgänge bei der Blutentnahme über Funkfernsteuerung von Hand ausgelöst werden können, ist es zweckmäßig, hierbei so vorzugehen, daß die Pumpe für eine bestimmte Zeit in Tätigkeit gesetzt wird, daß während eines ersten Zeitabschnittes die von der Pumpe angesaugte Körperflüssigkeit in den freien Abfluß gepumpt wird, daß dann die Pumpe stillgesetzt wird und ein Mehrwegeventil vor dem Vorratsbehälter derart verstellt wird, daß der im Schieber vorgesehene Kanal vor der Einflußöffnung in einen noch leeren Probeaufnahmeraum zu stehen kommt, daß die Pumpe dann wieder in Betrieb gesetzt wird und den Vorratsraum mit Körperflüssigkeit füllt, daß nach einem weiteren bestimmten Zeitabschnitt die Pumpe wieder außer Tätigkeit gesetzt wird, daß das Mehrwegeventil wieder verstellt wird und zwar so, daß der Kanal im Schieber mit dem freien Abfluß fluchtet und daß hieran anschließend eine bestimmte Zeit Spülmittel einerseits in das zum Mehrwegeventil führende Kanalsystem, andererseits in die Schlauchpumpe und in den Katheter eingeleitet wird. Hierdurch ist gesichert, daß in den Proberöhrchen sich lediglich die Blutproben und nicht ein Gemisch mit Spülmittelresten befindet.

Das Wesen der Erfindung ist nachstehend anhand von zwei iu der Zeichnung schematisch dargestellten Ausführungsbeispielen näher erläutert. Es zeigen:

Fig. 1 ein Entnahmegerät mit einem Vorratsbehälter, der viele einzelne Räume für die Aufnahme der Proben aufweist,

Fig. 2 ein Entnahmegerät mit einem schlauchförmigen Speicher.

Das Entnahmegerät ist in einem Gehäuse 1 untergebracht, welches einen Durchlaß oder Anschlußstutzen 2 für den Eintritt oder den Anschluß eines Dauerkatheters aufweist. Aus diesem Dauerkatheter wird Körperflüssigkeit über den Anschlußstutzen 2 von der Schlauchpumpe 3 angesaugt und über den Kanal 4, das Rückschlagventil 5 und den Kanal 6 einem Mehrwegeventil 7 zugeführt, welches in seinem Gehäuse 71 eine Vielzahl von Ausflußöffnungen 72, 73, 74, 75... aufweist, deren jede zweite 72, 74 zu verschiedenen Aufnahmebehältern 8, z. B. Reagenzgläsern, für die Aufnahme der Flüssigkeitsproben führen, während jede dazwischen liegende Aufnahmeöffnung 73, 75... in einen gemeinsamen freien Abfluß führt. Im Inneren des Mehrwegedrehventiles 7 ist ein Schieber 76 angeordnet, in welchem ein Kanal 77 vorgesehen ist, der den Kanal 6 mit jeweils einer der Ausflußöffnungen 72, 73, 74, 75... verbinden kann. Zweckmäßig ist der Schieber 7 ein Drehschieber mit zentraler Zuführung und peripherer Abführung, welche - je nach Ventilstellung mit jeweils einer Ausflußöffnung kommuniziert. Mindestens eine der Ausflußöffnungen im Gehäuse 71 führt in den Kanal 78 für den freien Abfluß der Spülflüssigkeit und für den Abfluß des Spülflüssigkeit-Körperflüssigkeit-Gemisches, welches mit dem Anfangstakt der Schlauchpumpe angesogen und durch das Kanalsystem 4, 6 zum Mehrwegedrehventil geführt wird.

Für die Spülung nach jeder einzelnen Körperflüssigkeitsentnahme ist ein Reservoir 9 vorgesehen, von welchem über das Magnetventil 10 eine Leitung 11 in den Kanal 4 führt. Das Reservoir 9 ist zweckmäßigerweise ein Flüssigkeitsspeicher, ausgebildet als gas bzw. luftfreier flexibler Behälter, der bei den fraktionierten Entnahmen der Spül- und Füllflüssigkeit in sich zusammenfällt. Dadurch ist gewährleistet, daß das gesamte Katheter-Schlauchsystem gas- bzw. luftleer bleibt. Die Entnahme der Spülflüssigkeit erfolgt nach Öffnung des Magnetventils über die Leitung 19 durch die Tätigkeit der Pumpe 3. Als Spülflüssigkeit bei Blutentnahme wird eine heparinisierte physiologische Kochsalzlösung verwendet. Das Magnetventil 10 und das Mehrwegedrehventil 7 werden durch eine Steuerungsvorrichtung 12 ebenso wie die Pumpe 3 betätigt. Die Tätigkeit dieser Steuerungsvorrichtung 12 wird durch den Empfänger 13 ausgelöst, dessen Antenne 14 Signale des Senders 15 auffängt. Die elektrische Versorgung des Empfängers 13 der Steuervorrichtung 12, des Magnetventiles 10 und der Schlauchpumpe 3 erfolgt aus einem Akkumulator 16.

Die Steuervorrichtung 12 wird durch einen Impuls aus dem Empfänger 13 in Tätigkeit gesetzt und läßt den Entnahmevorgang und den anschließenden Spülvorgang in verschiedenen einzelnen Schritten folgendermaßen ablaufen: Nach Empfang des die Steuerungsvorrichtung 12 in Tätigkeit setzenden Impulses wird die Schlauchpumpe 3 über die elektrische Leitung 17 bei geschlossenem Magnetventil 10 in Tätigkeit gesetzt. Hierdurch wird Flüssigkeit aus dem Dauerkatheter 2 in die Leitung 4 über das Rückschlagventil 5 in die Leitung 6 und von dort in das Mehrwegedrehventil 7 eingeleitet. Dieses steht in derjenigen Stellung, daß der Kanal 77 im Schieber 76 mit der Ausflußöffnung in den freien Abfluß 78 mündet. Hierdurch wird die vor der Entnahme im Dauerkatheter befindliche Spüllösung sowie ein Teil der angesaugten Körperflüssigkeit in den freien Abfluß geleitet (verworfen). Nach einer taktmäßig vorgegebenen Zeit wird von der Steuervorrichtung 12 ein weiterer Impuls über die elektrische Leitung 18 dem Motor des Mehrwegedrehventiles 7 zugeführt, wodurch dieser den Schieber 76 verdreht, und zwar so, daß der Kanal 77 im Drehschieber 76 vor der ersten zu einem Aufnahmeraum für die Probe führenden Ausflußöffnung stehenbleibt. Vor jeder Bewegung des Schiebers wird die Pumpe stillgesetzt und nach Erreichen der neuen Schieberposition wieder in Tätigkeit gesetzt. Das Drehventil ist während jeder Bewegung drucklos. Die Dichtungsflächen werden nicht belastet. Ist soviel Zeit verstrichen, daß sich der Aufnahmeraum 8 für die Aufnahme der Körperflüssigkeitsprobe ausreichend füllen konnte, wird von der Steuervorrichtung 12 ein weiterer Impuls abgegeben, welcher über die Leitung 18 den Motor des Mehrwegedrehventiles so bewegt, daß dieser vor die Ausflußöffnung 73 gelangt, die über den Kanal 79 mit der Leitung für den freien Abfluß der Spülflüssigkeit 78 verbunden ist. Weiter wirkt dieser Impuls über die Leitung 17 auf die Schlauchpumpe und setzt deren Tätigkeit still. Weiter wirkt dieser Impuls auch über die Leitung 19 auf das Magnetventil 10, welches kurzzeitig geöffnet wird und Spül- und Füllflüssigkeit über die Leitung 11 in den Kanal 4, das Rückschlagventil 6 und das Mehrwegedrehventil 7 einführt und gleichzeitig auch Spülflüssigkeit in die Schlauchpumpe und den Anschlußstutzen einführt. Nach einer bestimmten kurzen Zeitspanne schließt das Magnetventil wieder und das Gerät steht für den nächsten Entnahmevorgang zur Verfügung. Die exakte Stellung des Drehschiebers wird über eine Lichtschranke kontrolliert. Bei dem nächsten Entnahmevorgang wird dann zuerst der Drehschieber so mit seinem Kanal 77 vor der Ausflußöffnung stehenbleiben, daß im Dauerkatheter befindliche Spülflüssigkeit durch die Leitung 79 in die Leitung 78 einfließen und dadurch frei ausfließen kann. Bei diesem Entnahmevorgang wird dann der Drehschieber 76 so verdreht, daß er Kanal 77 vor die Ausflußöffnung 74 gerückt wird, um den nächsten Vorratsraum 8, der an diese Ausflußöffnung angeschlossen ist, zu füllen. So wird bei jeder Entnahme von Körperflüssigkeit ein anderer Vorratsraum 8 gefüllt. Der Motor des Mehrwegedrehventils kann ein Schrittschaltwerk sein, weil immer zwischen drei Kanälen, die zu zwei benachbarten Vorratsräumen 8 führen, ein Kanal angeordnet ist, der in den Kanal 78 für den freien Abfluß der Spülflüssigkeit führt.

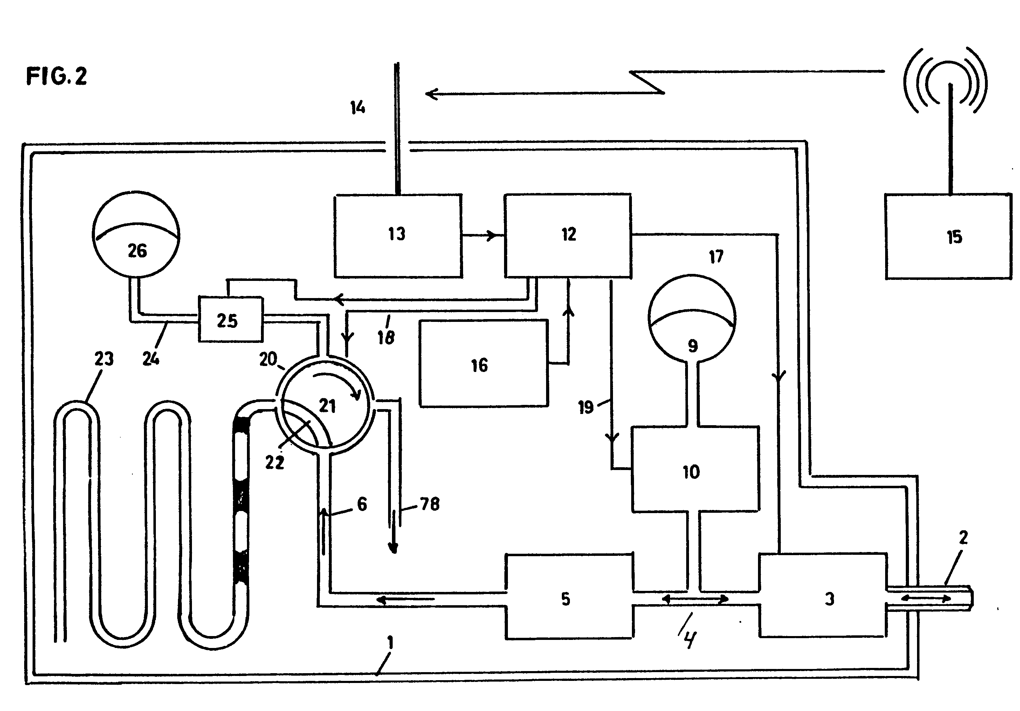

Das Ausführungsbeispiel der Fig. 2 unterscheidet sich wesentlich dadurch, daß anstelle des Mehrwegedrehventiles 7 ein Dreiwegeventil 20 vorgesehen ist, dessen Drehschieber 21 einen gekrümmten Kanal 22 aufweist. Weiter ist anstelle der verschiedenen Probenvorratsräume 8 ein Vorratsbehälter in Form eines Schlauches 23 getreten. Der Kanal 22 verbindet in einem ersten Schritt beim Entnahmevorgang den Kanal 6 mit der ins Freie führenden Abflußleitung 78, in einer zweiten Stellung beim zweiten Schritt den Kanal 6 mit dem Schlauch 23 und in einem dritten Schritt den Schlauch 23 über den Kanal 24 und das Magnetventil 25 mit dem Vorratsbehälter 26 für das Puffergas. Dieser Vorratsbehälter ist ein Druckbehälter, in welchem sich das Trenngas unter höherem Druck befindet.

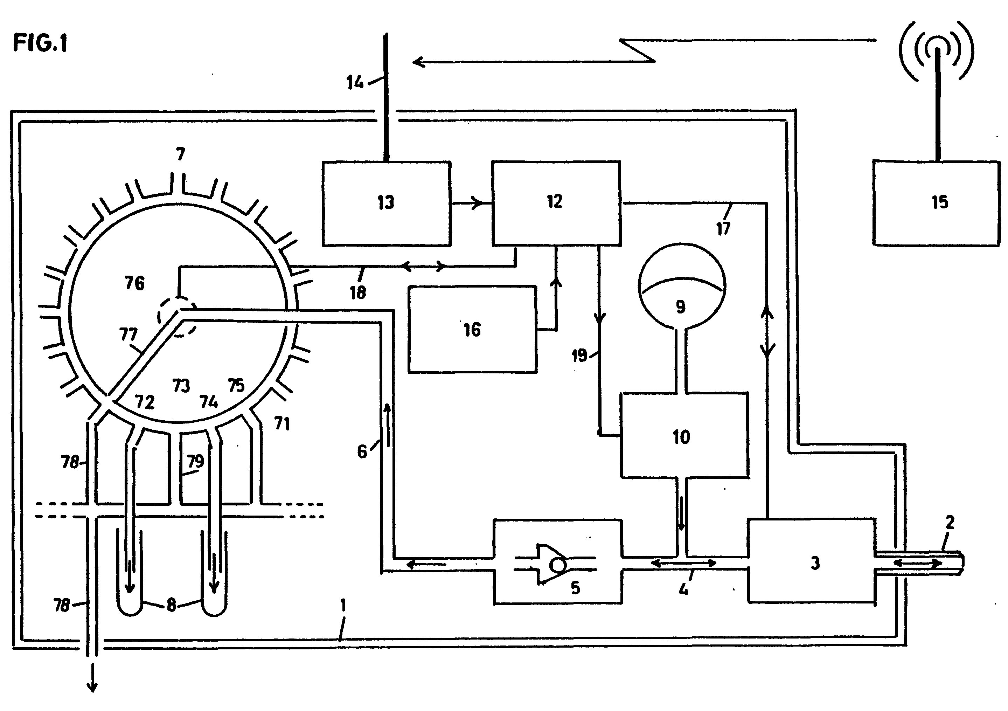

Die Steuerung dieser Entnahmevorrichtung erfolgt in ganz ähnlicher Weise wie die Steuerung der Fig. 1. Durch ein Schrittschaltwerk wird eine bestimmte Zeit nach Ingangsetzen der Vorrichtung der Drehschieber 21 aus der Stellung, in der der Kanal 22 den Kanal 6 mit der Abflußleitung 78 verbindet, in diejenige Stellung verschwenkt, in der der Kanal 6 mit dem Schlauch 23 verbunden wird. Nach einer weiteren Zeitspanne wird durch das Schrittschaltwerk der Drehschieber 21 wiederum um 90° derart verdreht, daß der Kanal 22 den Speicher 26 für das Trenngas mit dem Schlauch 23 verbindet. Kurzzeitig wird das Magnetventil 25 geöffnet, so daß eine bestimmte Menge Pufferflüssigkeit in den Schlauch 23 einfließen kann. Anschließend erfolgen zwei Schaltschritte nacheinander, so daß der Drehschieber 21 um zweimal 90° gedreht wird, wodurch der Kanal 22 im Drehschieber 21 wiederum den Kanal 6 mit der Abflußleitung 78 verbindet. Nun kann die Spülung mit der Spülflüssigkeit aus dem Reservoir erfolgen.

**Patentansprüche**

1. Entnahmegerät für Blut und andere Körperflüssigkeiten, bestehend aus einem Katheter, einer Pumpe (3), einem Vorratsbehälter (8), in welchem mehrere in zeitlichem Abstand gewonnene Körperflüssigkeitsproben getrennt speicherbar sind, einer Spül- und Füllvorrichtung (9, 10) für die Pumpe (3), den Katheter und ein von der Pumpe (3) zum Speicher (8) führendes Kanalsystem (4, 6), wobei ein Ventil (7) oder Ventilsystem für die Durchleitung der entnommenen Körperflüssigkeit und der Spül- und Füllflüssigkeit für das Kanalsystem (4, 6) vor dem Vorratsbehälter (8) angeordnet ist und einer Steuervorrichtung (12) für die Betätigung der Pumpe (3), der Spül- und Füllvorrichtung (9, 10) und dem Ventil (7) bzw. dem Ventilsystem, dadurch gekennzeichnet, daß das Entnahmegerät am Körper eines Probanden tragbar ausgestaltet ist und daß die Steuervorrichtung durch eine Fernsteuerung einschaltbar ist.

2. Entnahmegerät nach Anspruch 1, dadurch gekennzeichnet, daß der Vorratsbehälter mehrere Räume (8) für die Aufnahme der Proben aufweist, daß vor dem Vorratsbehälter ein Mehrwegeventil (7) angeordnet ist, bei dem je ein Weg zu je einem Raum (8) für die aufnahme einer Probe führt und daß im Mehrwegeventil (7) mindestens ein Weg (78, 79) für die Abführung der Spülflüssigkeit vorgesehen ist.

3. Entnahmegerät nach Anspruch 1, dadurch gekennzeichnet, daß das Mehrwegeventil (7) einen Schieber (76) mit einem Kanal (77) aufweist, dessen Mündung vor verschiedene Ausflußöffnungen (71, 72, 73, 74, 75...) einstellbar ist, von denen je eine zu verschiedenen Aufnahmebehältern und mindestens eine in einen freien Abfluß führt.

4. Entnahmegerät nach Anspruch 1,
dadurch gekennzeichnet,
daß der Vorratsbehälter aus einem Schlauch (23) oder Rohr besteht, in den bzw. das in ständigem Wechsel eine Körperflüssigkeitsprobe und eine Trenngasmenge nach jeder Betätigung der Pumpe (3) einleitbar ist,
daß ein Vorratsbehälter (26) für das Trenmgas vorgesehen ist,
daß ein Ventil (20) oder ein Ventilsystem vor dem Vorratsbehälter (23) angeordnet ist, mit dem nach jeder Körperflüssigkeitsprobe eine bestimmte Menge Trenngas in den Vorratsbehälter (23) einleitbar ist
und daß ein weiteres Ventil (10) oder Ventilsystem für die Einführung eines Spülmittels in das Kanalsystem (4, 6) zwischen der Pumpe (3) und dem Ventil (20) vor dem Vorratsbehälter (23) und in den Anschlußstutzen (2) vergesehen ist, wobei das Ventil (20) ver dem Vorratsbehälter (23) einen Weg (78) für die Abführung des Spülmittels aufweist.

5. Entnahmegerät nach Anspruch 1
dadurch gekennzeichnet,
daß die Fernsteuerung eine Funkfernsteuerung ist, die aus dem Sender (15) und dem Empfänger (13) besteht.

6. Entnahmegerät nach Anspruch 1,
dadurch gekennzeichnet,
daß die Steuervorrichtung für die Spülvorrichtung sowie für das dieser zugeordnete Ventil (10) und/oder die dem Vorratsbehälter (8, 23) zugeordneten Ventile (5, 7, 20) durch einen Impuls der Fernsteuerung in Tätigkeit setzbar ist
und daß die Steuervorrichtung mit einem Programm
a in den Abfall (78) pumpen
b umschalten
c in das Proberöhrchen (8) pumpen
d umschalten .
e spülen und in den Abfall laufenlassen
versehen ist.

7. Entnahmegerät nach Anspruch 1,
dadurch gekennzeichnet,
daß die Zuführungsleitung der Spülflüssigkeit zwischen dem Mehrwegeventil (7, 20) und der Pumpe (3) angeordnet ist,
wobei vorzugsweise die Pumpe (3) eine Schlauchpumpe ist
und wobei vorzugsweise der Spülflüssigkeitsbehälter ein flexiber gas- bzw, luftfreier Behälter ist, der bei fraktionierten Entnahmen in sich zusammenfällt.

8. Entnahmegerät nach Anspruch 1,
dadurch gekennzeichnet,
daß dessen Bauteile in einem Gehäuse (19) untergebracht sind, welches mit einem Anschnallgurt versehen ist.

9. Entnahmegerät nach Anspruch 1,
dadurch gekennzeichnet,
daß der Vorratsbehälter kühlbar ist und vorzugsweise die Vorratsräume oder -röhrchen in einem vorgekühlten Kühlblock untergebracht sind.

10. Verfahren zum Betreiben eines tragbaren Gerätes zur Entnahme von Blut und anderen Körperflüssigkeiten nach einem der vorhergehenden Ansprüche,
dadurch gekennzeichnet,
daß die Pumpe (3) für eine bestimmte Zeit in Tätigkeit gesetzt wird,
daß während eines ersten Zeitabschnittes die von der Pumpe angesaugte Körperflüssigkeit in den freien Abfluß gepumpt wird, daß dann die Pumpe stillgesetzt wird und ein Mehrwegeventil vor dem Vorratsbehälter derart verstellt wird, daß der im Schieber vorgesehene Kanal vor der Einflußöffnung in einen noch leeren Probeaufnahmeraum zu stehen kommt, daß die Pumpe dann wieder in Betrieb gesetzt wird und den Vorratsraum mit Körperflüssigkeit füllt, daß nach einem weiteren bestimmten Zeitabschnitt die Pumpe wieder außer Tätigkeit gesetzt wird,
daß das Mehrwegeventil wieder verstellt wird und zwar so, daß es in einer den Durchfluß erlaubenden Stellung befindlich ist, daß daran anschließend das Mehrwegeventil wieder so verstellt wird, daß der Kanal im Schieber mit dem freien Abfluß fluchtet,
und daß hieran anschließend eine bestimmte Zeit Spülmittel einerseits in das zum Mehrwegeventil führende Kanalsystem, andererseits in die Schlauchpumpe und in den Katheter eingeleitet wird.

**Revendications**

1. Appareil de prélèvement de sang et autres liquides du corps, consistant en un cathéter, une pompe (3), un réservoir (8), dans lequel plusieurs échantillons de liquide du corps obtenus à un certain intervalle de temps peuvent être introduits de façon séparée, un dispositif (9, 10) de rinçage et remplissage pour la pompe (3), le cathéter et un système de canaux conduisant de la pompe (3) au réservoir (8), une vanne (7) ou un système de vanne étant disposé en amont du réservoir (8) pour commander le passage du liquide du corps prélevé et liquide de rinçage et de remplissage pour le système de canaux (4, 6), et un dispositif (12) de commande pour l'actionnement de la pompe (3), du dispositif (9, 10) de rinçage et remplissage et de la vanne (7) ou du système de vanne respectivement, caractérisé en ce que l'appareil de prélèvement est conçu de façon à pouvoir être porté contre le corps d'un patient et en ce que le dispositif de commande peut être mis en marche au moyen d'une télécommande.

2. Appareil de prélèvement selon la revendication 1, caractérisé en ce que le réservoir présente plusieurs capacités (8) pour la réception des échantillons, en ce qu'en amont du réservoir est disposée une vanne (7) à plusieurs voies dans laquelle une voie respective conduit à chaque capacité (8) de réception d'un échantillon, et en ce que dans la vanne (7) à plusieurs voies est prévue au moins une voie (78, 79) d'évacuation du liquide de rinçage.

3. Appareil de prélèvement selon la revendication 1, caractérisé en ce que la vanne (7) à plusieurs voies présente un tiroir (76) avec un canal (77) dont l'embouchure peut être placée en regard de différents orifices d'écoulement (71, 72, 73, 74, 75...) dont certains conduisent chacun à l'un, respectif, de différents récipients et dont au moins l'un conduit à un écoulement libre.

4. Appareil de prélèvement selon la revendication 1, caractérisé en ce que le réservoir consiste en un tuyau souple (23) ou en un tube, dans lequel peuvent être introduits de façon constamment alternée un échantillon de liquide du corps et une certaine quantité de gaz de séparation après chaque actionnement de la pompe (3), en ce qu'il est prévu un réservoir (26) pour le gaz de séparation, en ce qu'une vanne (20) ou un système de vanne, au moyen duquel une quantité déterminée de gaz de séparation peut être introduite dans le réservoir (23) après chaque échantillon de liquide du corps, est placé en amont du réservoir (23), et en ce qu'une autre vanne (10) ou un autre système de vanne pour l'introduction d'un milieu de rinçage dans le système de canaux (4, 6) est prévu entre la pompe (3) et la vanne (20), en amont du réservoir (23), et dans les tubulures de jonction (2), la vanne (20) présentanit en amont du réservoir (23) une voie (78) pour l'évacuation du milieu de rinçage.

5. Appareil de prélèvement selon la revendication 1, caractérisé en ce que la télécommande est une radio-commande, qui consiste en l'émetteur (15) et le récepteur (13).

6. Appareil de prélèvement selon la revendication 1, caractérisé en ce que le dispositif de commande pour le dispositif de rinçage ainsi que pour la vanne (10) associée à celui-ci et/ou les vannes (5, 7, 20) associées au réservoir (8, 23) peut être mis en action au moyen d'une impulsion de la télécommande et en ce que le dispositif de commande est pourvu d'un programme pour:
    a) pomper vers l'évacuation (78),
    b) commuter,
    c) pomper vers le petit tube à échantillon (8),
    d) commuter,
    e) rincer et laisser s'écouler vers l'évacuation.

7. Appareil de prélèvement selon la revendication 1, caractérisé en ce que la conduite d'amenée du liquide de rinçage est disposée entre la vanne (7, 20) à plusieurs voies et la pompe (3), la pompe (3) étant de préférence une pompe péristaltique et le réservoir de liquide de rinçage étant de préférence un réservoir flexible ne contenant ni gaz, ni air, qui s'affaisse lors de prélèvements fractionnés.

8. Appareil de prélèvement selon la revendication 1, caractérisé en ce que ses constituants sont logés dans un boîtier (19) qui est pourvu d'une ceinture de fixation.

9. Appareil de prélèvement selon la revendication 1, caractérisé en ce que le réservoir peut être réfrigéré et en ce que, de préférence, les capacités ou petits tubes formant réservoir sont placés dans un bloc de réfrigération pré-refroidi.

10. Procédé de fonctionnement d'un appareil portatif pour le prélèvement de sang et autres liquides du corps, selon l'une des revendications précédentes, caractérisé en ce que la pompe (3) est mise en action pour un temps déterminé, en ce que, pendant un premier lapse de temps, le liquide du corps aspiré par la pompe est pompé vers l'écoulement libre, en ce qu'ensuite la pompe est arrêtée et une vanne à plusieurs voies, disposée en amont du récipient, est réglée de telle sorte que le canal prévu dans le tiroir vienne se placer devant un orifice d'écoulement vers un volume de réception d'échantillon encore vide, en ce que la pompe est alors remise en service et remplit le volume-réservoir en fluide du corps, en ce qu'après un autre lapse de temps déterminé, la pompe est à nouveau mise hors service, en ce que la vanne à plusieurs voies est à nouveau réglée, et ceci de telle sorte qu'elle se trouve dans une position permettant la traverssée, en ce qu'ensuite la vanne à plusieurs positions est à nouveau réglée de telle sorte que le canal du tiroir coïncide avec l'écoulement libre, et en ce qu'ensuite, pendant un temps déterminé, du milieu de rinçage est amené d'une part dans le système de canaux conduisant à la vanne à plusieurs voies et d'autre part dans la pompe péristaltique et dans le cathéter.

**Claims**

1. A device for withdrawing blood and other body fluids, consisting of a catheter, a pump (3), a storage container (8) in which a plurality of body fluid samples obtained at timed intervals can be separately stored, a flushing and filling device (9, 10) for the pump (3), the catheter and a channel system (4, 6) which leads from the pump (3) to the store (8), where a valve (7) or valve system for conducting the withdrawn body fluid and the flushing and filling liquid for the channel system (4, 6) is arranged in series with the storage container (8), and a control device (12) for the actuation of the pump (3), the flushing and filling device (9, 10) and the valve (7) or valve system,
    characterised in that the withdrawal device is designed to be carried on the person of an individual taking the sample;
    and that the control device can be switched on by a remote control device.

2. A withdrawal device as claimed in Claim 1, characterised in that the storage container comprises a plurality of chambers (8) which collect the samples;
    that the storage container is arranged in series with a multi-channel valve (7), in which each channel leads to a respective chamber (8) for the collection of a sample;

and that the multi-channel valve (7) includes at least one channel (78, 79) for the discharge of the flushing liquid.

3. A withdrawal device as claimed in Claim 1, characterised in that the multi-channel valve (7) comprises a slide (76) with a channel (77), the opening of which can be adjusted so as to align with different outlet openings (71, 72, 73, 74, 75...), each of which leads to different collecting containers, and at least one of which leads into a free drain.

4. A withdrawal device as claimed in Claim 1, characterised in that the storage container consists of a hose (23) or pipe into which, in continuous alternation, a body fluid sample and a quantity of separating gas can be introduced following each actuation of the pump (3);

that a storage container (26) is provided for the separating gas; that a valve (20) or valve system is arranged in series with the storage container (23), by which, following each body fluid sample, a specific quantity of separating gas can be introduced into the storage container (23);

and that a further valve (10) or valve system which introduces a flushing agent into the channel system (4, 6) between the pump (3) and the valve (20) is arranged in series with the storage container (23) and in the connection pieces (2), the valve (20) in series with the storage container (23) being provided with a channel (78) for the discharge of the flushing agent.

5. A withdrawal device as claimed in Claim 1, characterised in that the remote control device is a radio remote control device which consists of the transmitter (15) and the receiver (13).

6. A withdrawal device as claimed in Claim 1, characterised in that the control device for the flushing device and for the valve (10) assigned thereto, and/or the valves (5, 7, 20) assigned to the storage container (8, 23) can be activated by a pulse from the remote control device;

and that the control device is provided with the programme:

a pump into the drain (78)
b switch-over
c pump into the sample pipe (8)
d switch-over
e flush and discharge into the drain.

7. A withdrawal device as claimed in Claim 1, characterised in that the supply line for the flushing liquid is arranged between the multi-channel valve (7, 20) and the pump (3),

where the pump (3) is preferably a peristaltic pump and where the flushing liquid container is preferably a flexible gas-free and air-free container which collapses in the case of fractional withdrawals.

8. A withdrawal device as claimed in Claim 1, characterised in that the components thereof are accommodated in a housing (19) which is provided with a safety belt.

9. A withdrawal device as claimed in Claim 1, characterised in that the storage container can be cooled and the storage chambers or pipes are preferably accommodated in a pre-cooled cooling block.

10. A method of operating a portable device for the withdrawal of blood and other body fluids, as claimed in one of the preceding Claims, characterised in that the pump (3) is activated for a specific length of time;

that during a first time interval the body fluid sucked in by the pump is pumped into the free drain;

that the pump is then stopped and a multi-channel valve arranged in series with the storage container is adjusted in such manner that the channel arranged in the slide comes into alignment with the inlet opening into an as yet empty sample-collecting chamber;

that the pump is then re-started and fills the storage chamber with body fluid;

that after a further specified time interval, the pump is stopped again;

that the multi-channel valve is re-adjusted in such manner that it occupies a position which permits a through-flow;

that then the multi-channel valve is again re-adjusted in such manner that the channel in the slide aligns with the free drain; and that then, for a specified interval of time, flushing agent is introduced, on the one hand, into the channel system which leads to the multi-channel valve and, on the other hand, into the peristaltic pump and the catheter.

FIG.1

0 139 706

FIG.2